# EUROPEAN PATENT APPLICATION

(11) **EP 2 200 405 A2**
(43) Date of publication of application: **23.06.2010**
(21) Application number: 09175955.5
(22) Date of filing: 13.11.2009
(51) Int. Cl.: H05B 33/08

(54) **Tracking regulator for LED load and power regualtion method thereof**

(30) Priority: 10.12.2008 TW 97222188 U
(71) Applicant: Sinergy Power Solution Inc., Taipei 10053 (TW); Sinergy Micro Devices Inc., Keelung City 205 (TW); Fang-Wei, Lee, Wenshan Dist., Taipei City 116 (TW)
(72) Inventor: Lee, Fang-Wei, Wenshan Dist., Taipei City 116 (TW)
(74) Representative: 2K Patentanwälte Blasberg Kewitz & Reichel

(57) **Abstract**

A tracking regulator for a plurality of light emitting diode (LED) loads is provided. The tracking regulator includes a power supply and a control circuit coupled to the power supply. The power supply provides a power signal to each of the LED loads according to a control signal, wherein each of the LED loads provides an output signal according to the power signal. The control circuit detects the output signals from the LED loads to generate the control signal, so as to control the power supply to adjust the power signal.

## Description

### CROSS REFERENCE TO RELATED APPLICATIONS

This Application claims priority of Taiwan Patent Application No. 097222188, filed on December 10, 2008, the entirety of which is incorporated by reference herein.

### BACKGROUND OF THE INVENTION

### Field of the Invention

The invention relates to a tracking regulator, and more particularly to a tracking regulator which provides optimum power to a plurality of light emitting diode (LED) loads.

### Description of the Related Art

Light emitting diode (LED) lighting systems are used as a source for general lighting applications. In the last twenty years, rapid commercialization of LED devices has occurred. LED lighting systems are applied in hand-held flashlights, desk lamps and so on. Additionally, high-powered LEDs are being applied to large-scale outdoor lighting systems. Moreover, LEDs are also widely applied in backlight modules.

In general, power supply for an LED of LED lighting systems or LED backlight modules, may output power at a fixed voltage. However, forward voltage of LEDs may be unstable or different due to process variability or temperature. Accordingly, as an example, a power supply may provide insufficient current to drive the LEDs, thus decreasing brightness of the LEDs or electron-to-photon conversion efficiency of the lighting systems or backlight modules utilizing the LEDs. In order to solve aforementioned problem, a power supply may provide a high current to drive the LEDs. However, in this case, power consumption and light attenuation would increase, thereby decreasing operating lifespan of the LEDs.

Therefore, a tracking regulator for a plurality of LED loads and a power regulation method thereof are desired to obtain optimum conversion efficiency and decrease power consumption.

### BRIEF SUMMARY OF THE INVENTION

Tracking regulator and power regulation method for a plurality of LED loads are provided. An exemplary embodiment of such a tracking regulator comprises a power supply and a control circuit coupled to the power supply. The power supply provides a power signal to each of the LED loads according to a control signal, wherein each of the LED loads provides an output signal according to the power signal. The control circuit detects the output signals from the LED loads to generate the control signal, so as to control the power supply to adjust the power signal.

Furthermore, an exemplary embodiment of a power regulation method for providing a power signal to a plurality of LED loads is provided. Output signals of the LED loads are detected by a control circuit so as to generate a control signal. Voltage or current of the power signal is adjusted according to the control signal by a power supply.

A detailed description is given in the following embodiments with reference to the accompanying drawings.

### BRIEF DESCRIPTION OF DRAWINGS

The invention can be more fully understood by reading the subsequent detailed description and examples with references made to the accompanying drawings, wherein:

FIG. 1 shows a tracking regulator for a plurality of LED loads according to an embodiment of the invention;

FIG. 2 shows a waveform diagram illustrating variation of the current Ia of FIG. 1;

FIG. 3 shows a schematic illustrating a tracking regulator with a plurality of control circuits according to an embodiment of the invention;

FIG. 4 shows a tracking regulator according to another embodiment of the invention; and

FIG. 5 shows a power regulation method for providing a power signal to a plurality of LED loads according to an embodiment of the invention.

### DETAILED DESCRIPTION OF THE INVENTION

The following description is of the best-contemplated mode of carrying out the invention. This description is made for the purpose of illustrating the general principles of the invention and should not be taken in a limiting sense. The scope of the invention is best determined by reference to the appended claims.

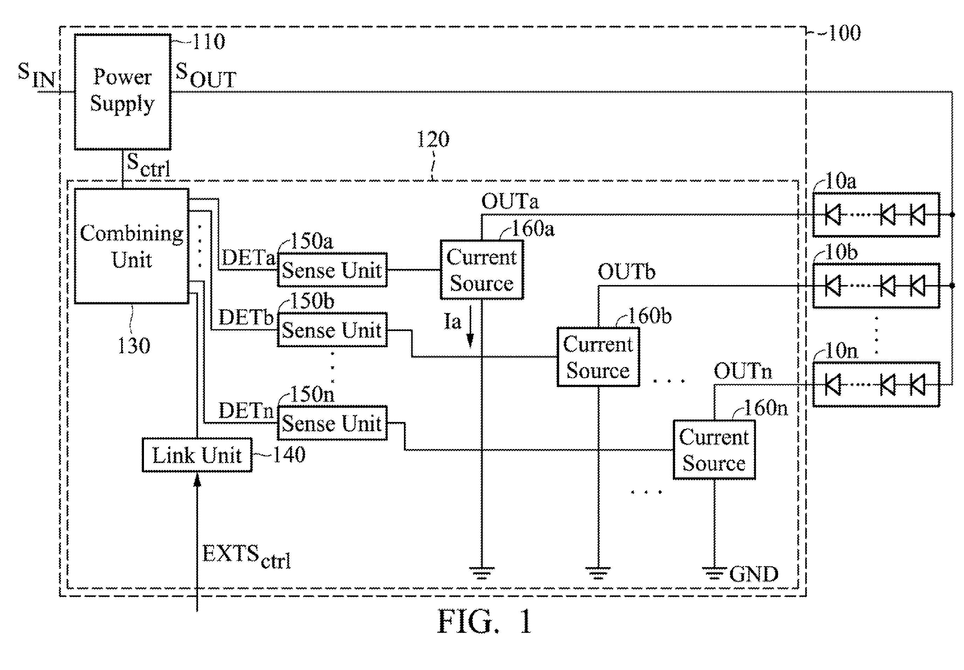

FIG. 1 shows a tracking regulator 100 for a system with a plurality of LED loads 10a-10n according to an embodiment of the invention. The tracking regulator 100 comprises a power supply 110 and a control circuit 120. The power supply 110 is a power converter, which may be an AC to DC or DC to DC power supply. The power supply 110 receives and converts an input power signal S_{IN} to output an output power signal S_{OUT}, and the power supply 110 further controls the voltage or current of the output power signal S_{OUT} according to a control signal S_{ctrl} from the control circuit 120. In addition, the power supply 110 provides the power signal S_{OUT} to drive the LED loads 10a-10n, wherein each of the LED loads 10a-10n comprises a plurality of LED diodes connected in series. When the power signal S_{OUT} is applied to the LED loads 10a-10n, each LED loads 10a-10n may provide an output signal, such as OUTa, OUTb or OUTn to a corresponding current source. Taking the LED load 10a as an example, the output signal OUTa thereof may be a current flowing through the LED load 10a or a voltage corresponding to the current of the LED load 10a.

In FIG. 1, the control circuit 120 comprises the current sources 160a-160n, the sense units 150a-150n, a combining unit 130 and a link unit 140. Each current source is coupled between a corresponding LED load and a ground GND, which is used to drain a current from the corresponding LED load according to the output signal of the corresponding LED load. For example, the current source 160a coupled to the LED load 10a may drain a current Ia from the LED load 10a according to the output signal OUTa. In one embodiment, the current source 160a further limits the magnitude of the current Ia flowing through the LED load 10a. Each sense unit is coupled to a corresponding current source, which is used to sense/detect the current drained by the corresponding current source and to generate a detect signal according to the sensed current. Taking the sense unit 150a as an example, the sense unit 150a is coupled to the current source 160a for sensing the current Ia drained by the current source 160a. After sensing the current Ia, the sense unit 150a may provide a detect signal DETa to indicate whether the current Ia is larger than or equal to a first current I1 (i.e. Ia≧I1) or the current Ia is smaller than a second current I2 (i.e. Ia<I2). Specifically, the detect signal (e.g. DETa, DETb or DETn) is changed to a first voltage level when the drained current of the corresponding current source (e.g. 160a, 160b or 160n) is larger than or equal to the first current I1, and the detect signal is changed to a second voltage level when the drained current of the corresponding current source is smaller than the second current I2. After receiving all of the detect signals DETa-DETn, the combining unit 130 generates the control signal S_{ctrl} to the power supply 110 to adjust the voltage or current of the power signal S_{OUT} when the link unit 140 is disabled.

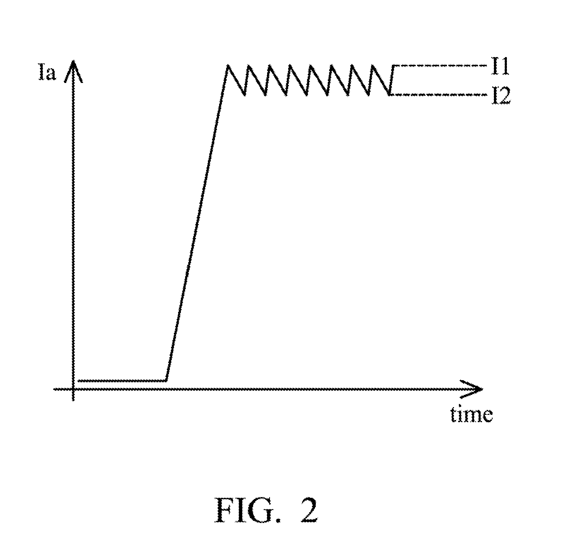

The first current I1 and the second current I2 are determined according to desired brightness, operating voltage or current of the LED loads. Due to process variability, characteristics of the LED diodes within the LED loads may be different. Thus, the brightness of the LED loads under the same operating conditions or the operating voltage/current of the LED loads are varied. In one embodiment, the first current I1 is a current indicating that the power signal S_{OUT} is high enough to ensure that operation or brightness of any LED load is normal, and the second current I2 is a current indicating that the power signal S_{OUT} is to low to ensure that the operation or brightness of one of the LED loads is normal, wherein the first current I1 is larger than the second current I2. Therefore, when the received detect signals DETa-DETn indicate that each of the drained currents is larger than or equal to the first current I1, the combining unit 130 generates the control signal S_{ctrl} to the power supply 110 so that the power supply 110 decreases the current or voltage of the power signal S_{OUT}, thus avoiding unnecessary power consumption caused by the power signal S_{OUT} with higher current or voltage. On the contrary, the combining unit 130 generates the control signal S_{ctrl} to the power supply 110 so that the power supply 110 increases the current or voltage of the power signal S_{OUT} when the received detect signals DETa-DETn indicate that one of the drained currents is smaller than the second current I2, thus assuring that the operation or brightness of any LED load of the system is normal. In another embodiment, the first current I1 is identical to the second current I2 in order to simplify circuit design. Referring to FIG. 2, FIG. 2 shows a waveform diagram illustrating variation of the current Ia of FIG. 1. It is notable that the current Ia is changed between the first current I1 and the second current I2.

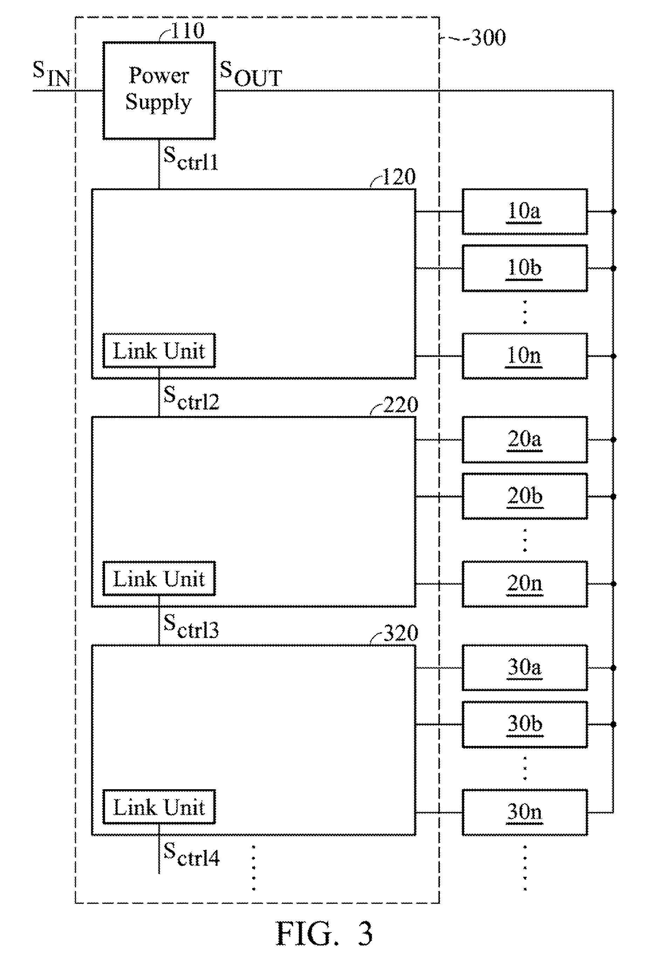

In FIG.1, the link unit 140 is an interface for integrating an external control signal EXTS_{ctrl} provided by another control circuit. Therefore, the combining unit 130 generates the control signal S_{ctrl} according to the external control signal EXTS_{ctrl} when the link unit 140 is enabled. FIG. 3 shows a schematic illustrating a tracking regulator 300 with a plurality of control circuits according to an embodiment of the invention. The tracking regulator 300 comprises the power supply 110, the control circuit 120 coupled to the LED loads 10a-10n, a control circuit 220 coupled to the LED loads 20a-20n, a control circuit 320 coupled to the LED loads 30a-30n and so on. In the embodiment, by connecting the plurality of control circuits in series, the tracking regulator 300 may drive more LED loads than the tracking regulator 100 of FIG. 1. As described above, the control circuit 320 provides a control signal S_{ctrl3} to the control circuit 220 according to the output signals of the LED loads 30a-30n and a control signal S_{ctrl4} from a next control circuit of the control circuit 320, and the control circuit 220 provides a control signal S_{ctrl2} to the control circuit 120 according to the output signals of the LED loads 20a-20n and the received control signal S_{ctrt3}. The control circuit 120 provides a control signal S_{ctrl1} to the power supply 110 according to the output signals of the LED loads 10a-10n and the received control signal S_{ctrl2}, so as to control the voltage or current of the power signal S_{OUT}. Accordingly, the tracking regulator 300 may provide the power signal S_{OUT}, operating at a specific voltage or current range, to drive all of the LED loads, thus avoiding unnecessary power consumption caused by the power signal S_{OUT} with higher than required current or voltage. In one embodiment, the control circuits are implemented in different integrated circuit (IC).

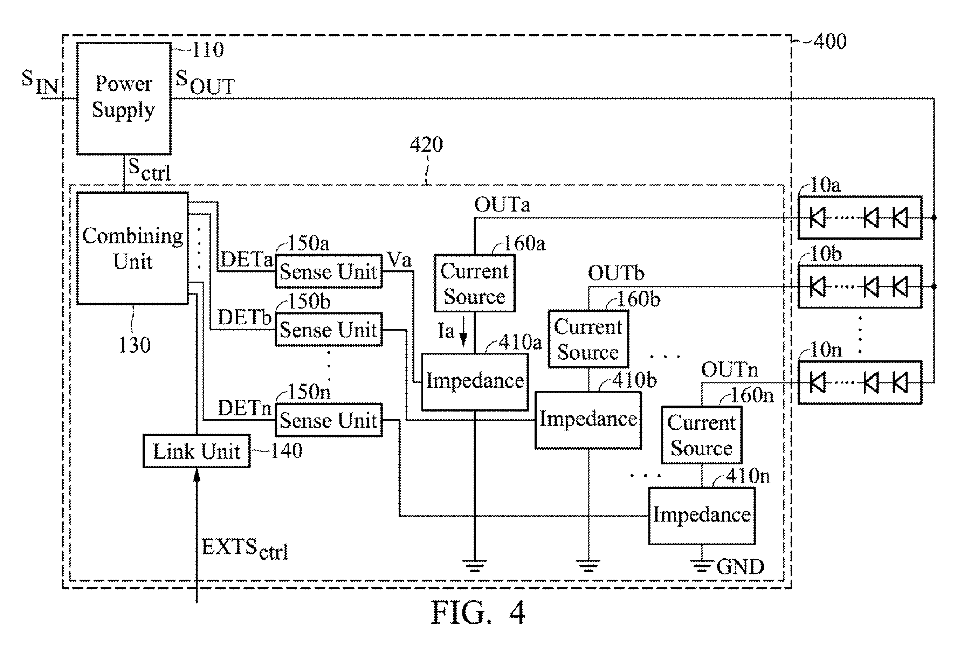

FIG. 4 shows a tracking regulator 400 according to another embodiment of the invention. The tracking regulator 400 comprises the power supply 110 and a control circuit 420. Compared with the control circuit 120 of FIG. 1, the control circuit 420 further comprises the impedance units 410a-410n, wherein each of the impedance units 410a-410n is a device with resistance and is coupled to a corresponding current source. In FIG. 4, each of sense units 150a-150n is coupled to a corresponding impedance unit, which is used to sense/detect a voltage of the corresponding impedance unit and to generate a detect signal according to the sensed voltage, wherein the sensed voltage corresponds to the current drained by the corresponding current source. For example, the sense unit 150a is coupled to the impedance unit 410a and the sense unit 150a senses a voltage Va corresponding to the current Ia. After sensing the voltage Va, the sense unit 150a provides the detect signal DETa to indicate whether the voltage Va is larger than or equal to a first voltage V1 (i.e. Va≧V1) or the voltage Va is smaller than a second voltage V2 (i.e. Va<V2). The detect signal (e.g. DETa, DETb or DETn of FIG. 4) is changed to a first voltage level when the sensed voltage of the impedance unit (e.g. 410a, 410b or 410n) is larger than or equal to the first voltage V1, and the detect signal is changed to a second voltage level when the sensed voltage of the impedance unit is smaller than the second voltage V2, wherein the first voltage V1 and the second voltage V2 are also determined according to desired brightness, operating voltage or current of the LED loads. Similarly, after receiving all of the detect signals DETa-DETn, the combining unit 130 generates the control signal S_{ctrl} to the power supply 110 to adjust the voltage or current of the power signal S_{OUT} when the link unit 140 is disabled. Therefore, when the received detect signals DETa-DETn indicate that each of the sensed voltages is larger than or equal to the first voltage V1, the combining unit 130 generates the control signal S_{ctrl} to the power supply 110 so that the power supply 110 decreases the current or voltage of the power signal S_{OUT}, thus avoiding unnecessary power consumption caused by the power signal S_{OUT} with higher than required current or voltage. On the contrary, the combining unit 130 generates the control signal S_{ctrl} to the power supply 110 to increase the current or voltage of the power signal S_{OUT} when the received detect signals DETa-DETn indicate that one of the sensed voltages is smaller than the second voltage V2, thus assuring that the operation or brightness of any LED load is normal. In another embodiment, the first voltage V1 is identical to the second voltage V2 in order to simplify circuit design. Similarly, by connecting the plurality of control circuits 420 in series, the tracking regulator 400 may drive more LED loads.

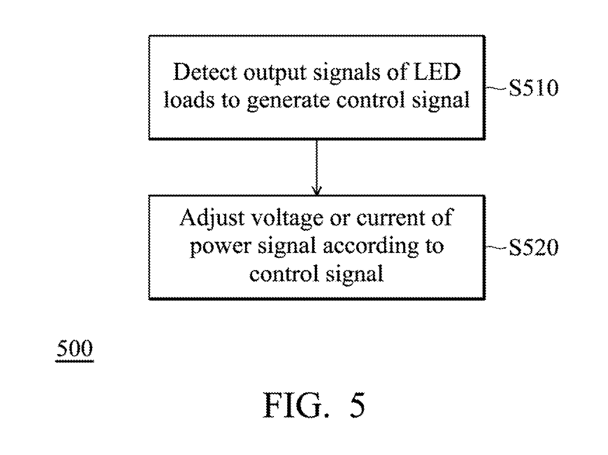

FIG. 5 shows a power regulation method 500 for providing a power signal to a plurality of LED loads according to an embodiment of the invention. First, a control circuit (e.g. 120 of FIG. 1 or 420 of FIG. 4) detects output signals from the plurality of LED loads to generate a control signal (step S510). Next, a power supply (e.g. 110 of FIG. 1 or FIG. 4) adjusts the voltage or current of the power signal according to the control signal (step S520). As described above, the power supply decreases the current or voltage of the power signal when the control signal indicates that current or voltage of each of the output signals is larger than or equal to a first specific value, and the power supply increases the current or voltage of the power signal when the control signal indicates that the current or voltage of one of the output signals is smaller than a second specific value. The first specific value is larger than or equal to the second specific value. Furthermore, the output signals of the LED loads may be a plurality of currents each flowing through a respective LED load or a plurality of voltages respectively corresponding to the currents.

While the invention has been described by way of example and in terms of preferred embodiment, it is to be understood that the invention is not limited thereto. Those who are skilled in this technology can still make various alterations and modifications without departing from the scope and spirit of this invention. Therefore, the scope of the present invention shall be defined and protected by the following claims and their equivalents.

## Claims

1. A tracking regulator for a plurality of light emitting diode (LED) loads, comprising:
a power supply, providing a power signal to each of the LED loads according to a first control signal, wherein each of the LED loads provides an output signal according to the power signal; and
a first control circuit coupled to the power supply, detecting the output signals from the LED loads to generate the first control signal, so as to control the power supply to adjust the power signal.

2. The tracking regulator as claimed in claim 1, wherein the first control circuit controls the power supply to decrease current or voltage of the power signal when current or voltage of each of the output signals is larger than or equal to a first specific value, and the first control circuit controls the power supply to increase the current or voltage of the power signal when the current or voltage of one of the output signals is smaller than a second specific value, wherein the first specific value is larger than or equal to the second specific value.

3. The tracking regulator as claimed in claim 1, wherein the first control circuit comprises:
a plurality of current sources, each coupled between a respective one of the LED loads and a ground for draining a current from the respective one of the LED loads;
a plurality of sense units, each coupled to a respective one of the current sources for sensing the drained current of the respective one of the current sources to generate a detect signal; and
a combining unit coupled to the sense units, generating the first control signal according to the detect signals from the sense units.

4. The tracking regulator as claimed in claim 3, wherein the combining unit generates the first control signal to the power supply to decrease current or voltage of the power signal when the detect signals indicate that each of the sensed currents is larger than or equal to a first current, and the combining unit generates the first control signal to the power supply to increase the current or voltage of the power signal when the detect signals indicate that one of the sensed currents is smaller than a second current, wherein the first current is larger than or equal to the second current.

5. The tracking regulator as claimed in claim 1, wherein the first control circuit comprises:
a plurality of current sources, each coupled to a respective one of the LED loads for draining a current from the respective one of the LED loads;
a plurality of impedance units, each coupled between a respective one of the current sources and a ground;
a plurality of sense units, each coupled to a respective one of the impedance units for sensing a voltage of the respective one of the impedance units to generate a detect signal; and
a combining unit coupled to the sense units, generating the first control signal according to the detect signals from the sense units.

6. The tracking regulator as claimed in claim 5, wherein the combining unit generates the first control signal to the power supply to decrease current or voltage of the power signal when the detect signals indicate that each of the sensed voltages is larger than or equal to a first voltage, and the combining unit generates the first control signal to the power supply to increase the current or voltage of the power signal when the detect signals indicate that one of the sensed voltages is smaller than a second voltage, wherein the first voltage is larger than or equal to the second voltage.

7. The tracking regulator as claimed in claim 2, further comprising:
a second control circuit coupled to the first control circuit, detecting the output signals from a portion of the LED loads to generate a second control signal,
wherein the first control circuit generates the first control signal according to the second control signal and the output signals from the other portion of the LED loads.

8. The tracking regulator as claimed in claim 7, wherein the first control circuit comprises:
a plurality of current sources, each coupled to a respective one of the other portion of the LED loads for draining a current from the respective one of the other portion of the LED loads;
a plurality of sense units, each coupled to a respective one of the current sources for sensing the drained current of the respective one of the current sources or sensing a voltage corresponding to the drained current to generate a detect signal;
a link unit coupled to the second control circuit, receiving the second control signal; and
a combining unit coupled to the sense units and the link unit, generating the first control signal according to the second control signal and the detect signals from the sense units.

9. The tracking regulator as claimed in claim 1, wherein each of the LED loads comprises a plurality of LED diodes connected in series, and the power supply is a power converter.

10. A power regulation method for providing a power signal to a plurality of LED loads, comprising:
detecting output signals of the LED loads to generate a control signal by a control circuit; and
adjusting voltage or current of the power signal according to the control signal by a power supply.

11. The power regulation method as claimed in claim 10, wherein the step of adjusting the voltage or current the power signal further comprises:
decreasing the current or voltage of the power signal when the control signal indicates that current or voltage of each of the output signals is larger than or equal to a first specific value; and
increasing the current or voltage of the power signal when the control signal indicates that the current or voltage of one of the output signals is smaller than a second specific value,
wherein the first specific value is larger than or equal to the second specific value.

12. The power regulation method as claimed in claim 10, wherein the step of detecting the output signals further comprises:
sensing a plurality of currents, each flowing through a respective one of the LED loads; and
generating the control signal according to the sensed currents.

13. The power regulation method as claimed in claim 12, wherein the control circuit controls the power supply to decrease current or voltage of the power signal when each of the sensed currents is larger than or equal to a first current, and the control circuit controls the power supply to increase the current or voltage of the power signal when one of the sensed currents is smaller than a second current, wherein the first current is larger than or equal to the second current.

14. The power regulation method as claimed in claim 10, wherein the step of detecting the output signals further comprises:
sensing voltages of a plurality of impedance units, wherein each of the impedance units is coupled between a respective one of the LED loads and a ground; and
generating the control signal according to the sensed voltages.

15. The power regulation method as claimed in claim 14, wherein the control circuit controls the power supply to decrease current or voltage of the power signal when each of the sensed voltages is larger than or equal to a first voltage, and the control circuit controls the power supply to increase the current or voltage of the power signal when one of the sensed voltages is smaller than a second voltage, wherein the first voltage is larger than or equal to the second voltage.
